# EUROPEAN PATENT APPLICATION

(11) **EP 0 922 439 A2**
(43) Date of publication of application: **16.06.1999**
(21) Application number: 98123553.4
(22) Date of filing: 10.12.1998
(51) Int. Cl.: A61C 8/00, A61B 17/86

(54) **Improved screw for intraosseus implant**

(30) Priority: 12.12.1997 IT MI970891 U
(71) Applicant: Faraci, Francesco, 21100 Varese (IT); L.S.S. LIFE SUPPORT SYSTEMS S.P.A., 07026 Olbia (Sassari) (IT)
(72) Inventor: Faraci, Francesco, 21100 Varese (IT)
(74) Representative: Cicogna, Franco

(57) **Abstract**

The present invention relates to an improved screw for an intraosseous implant, comprising a truncated cone collet connecting the stem and head of the screw. The threaded portion of the screw is moreover provided, along its helical extension, with three longitudinal slots which are arranged at 120° from one another, with respect to a plane perpendicular to the axis of the screw. The first part of the threaded portion, as considered starting from the truncated cone collet, is a continuous part.

## Description

### BACKGROUND OF THE INVENTION

The present invention relates to an improved screw for an intraosseous implant.

Conventional intraosseous implant to be implanted at a comparatively high depth, usually comprise a screw, provided with a screw head, and a screw stem, provided with a partially threaded portion.

The head of the screw is provided with an inner acetabulum, coaxial with the longitudinal axis of the screw and so shaped as to receive tools for screwing the screw into a pre-formed osseous cavity, and to allow a firm fitting of the stub on which the dental prosthesis will be formed.

One of the most important reasons of failures of an intraosseous implant of the above mentioned type, is an uneven distribution of occlusion loads through the bone structure in which the implant is received.

Actually, the mandibular bone construction is not homogeneous, but its mechanical features change radially from the surface to the inside.

The mandibular bone, as known, comprise a first high density layer (cortex bone), having good mechanical features (i.e. a good pressure resistance and resilient or elastic modulus).

This construction is even through a thickness of about 2.3 - 3.5 mm starting from the surface of the bone ridge.

This first layer is followed by a second transition layer, having a thickness of about 1 mm, therethrough the mechanical characteristics of the cortex bone are degraded, so as to gradually assume a more porous or fibrous construction.

In the third inner layer (sponge or medullar bone), having a comparatively large volume, the bone has a fibrous-lamellar characteristic, with great hollow grid spaces.

The mechanical features of this third bone layer are acceptable with respect to a mass holding of the implant (in fact, the helical portion of the implant is herein fitted), but the mechanical properties thereof are substantially insufficient to support the comparatively high pressing forces generated as the implant is subjected to occlusal loads.

In this connection it should be pointed out that prior implants of the above mentioned type conventionally use as structural material, a titanium material.

This material has physical (density) and mechanical (breaking load and resilient modulus) properties much better (from one to three orders) than those of the most compact cortex bone.

From this standpoint, the prior implants have a substantially isotropic construction, retransmitting to the encompassing environment the forces to which they are subjected without reducing or absorbing said forces in any substantial proportions.

In particular, the load is longitudinally concentrated in an annular region, having a limited surface and immediately overlaying a weaker bone construction.

A further drawback, caused by implants having steep diameter variations, is the generation of bone microcrackings due to the share force provided by the outer crown of the bottom base of the neck on the underlaying bone and/or due to an excessive concentration, greater than the resilient limit of the bone, of the longitudinal load in this area.

Both the above mentioned drawbacks can cause a secondary bone reabsorbing phenomenon, with a consequent weaking or loosing of the implant.

Thus, from the above discussion, it should be apparent that a need is at present felt of an improved screw for intraosseous implants allowing to overcome or greatly reduce the above mentioned drawbacks.

### SUMMARY OF THE INVENTION

Accordingly, the aim of the present invention is to provide a screw for intraosseous implants which is specifically designed for redistributing, in an optimum manner, to the encompassing bone construction, the pressing or compression loads.

Within the scope of the above mentioned aim, a main object of the present invention is to provide such a screw for intraosseous systems improving the stability properties of the implant in the time.

According to one aspect of the present invention, the above aim and objects are achieved by an improved screw for intraosseous implants, said screw comprising a head and a stem including a threaded portion, characterized in that said screw further comprises a truncated cone collet coupling said stem and head, and that said threaded portion is provided, along its helical extension, with a plurality of gaps.

According to a preferred embodiment of the invention, the threaded portion is provided with three longitudinal slots, arranged at 120° from one another, with respect to a plane perpendicular to the axis of said screw.

According to a further preferred embodiment of the invention, the threaded portion is provided with a continuous volute.

Finally, the improved screws for intraosseous implants according to the present invention is preferably made of titanium.

Thus, the aim and object of the invention are fully achieved, thereby the invention provides the following great advantages with respect to the prior art.

Firstly, the truncated cone configuration of the collet and the provision of a screw head having a diameter greater than that of the screw stem provide a greater stability of the screw, the diameter and length being the same, than conventional prior screws.

Moreover, the tapering configuration of the collet will radially discharge also that portion of the force rigid with the longitudinal axis of the screw, (i.e. the force of higher amplitude), deriving from a decomposition thereof according to the force parallelogram rules.

Thus, the gradual passage from the diameter to the collet to the stem of the screw will provide a radial load on a bone mass of a comparatively large amount, instead of longitudinal concentrating the load on an annular area, having a small surface and immediately overlaying a weaker bone construction.

Moreover, the longitudinal slots provide an essential function of allowing the formation of corresponding "pillars" providing a better joining of the bone sections filling the cavities or gaps separating the helix threads and the encompassing bone matrix.

The formation of these "pillars", moreover, will allow to absorb and redistribute the pressure occlusal load which is greatly concentrated on the first top turn or volute of a not slotted screw.

The first "volute" or turn of the screw thread, having a continuous circumferential construction, will allow to operate, at the medullar bone level, as an auxiliary means allowing the resilient absorbing of the occlusal load, as the implant bottom, as it frequently occurs, does not perfectly bear on a cortex matrix.

Moreover, the first volute will operate, during the stabilization period, as a plug element with respect to the underlaying cavities left by the longitudinal millings and with respect to those generated during the surgical application procedure.

### BRIEF DESCRIPTION OF THE DRAWINGS

Further advantages and characteristics of the present invention will become more apparent from the following disclosure, given by way of an illustrative but not limitative example, with reference to the accompanying drawings, where:

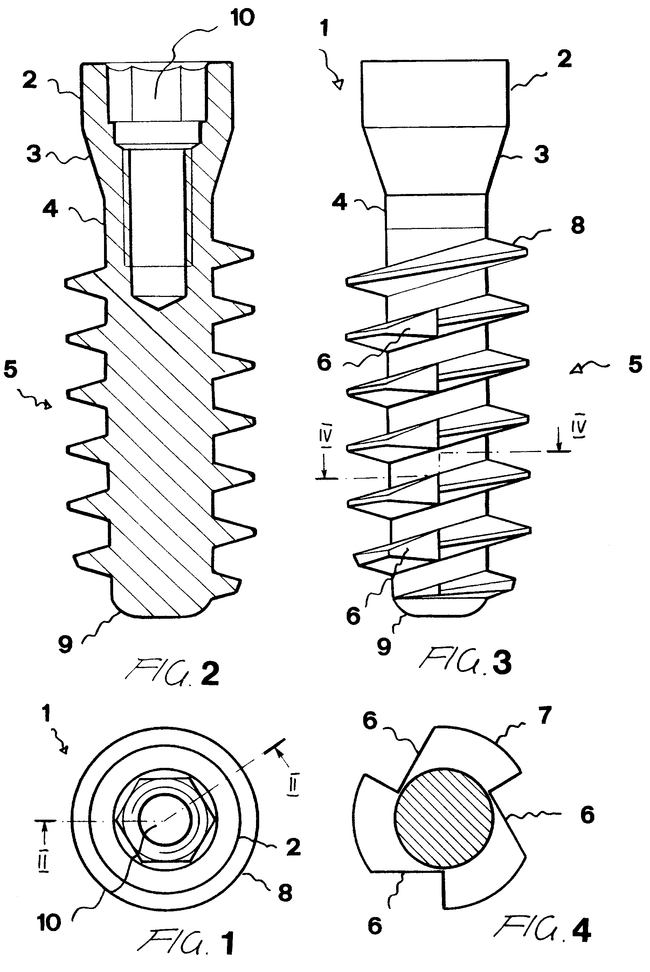

Figure 1 is a top plan view of the screw for intraosseous implants according to the present invention;
Figure 2 is a cross sectional view, taken substantially along the cross section line II-II, of Figure 1, and showing the intraosseous screw according to the present invention;
Figure 3 is a front view of the screw shown in Figures 1 and 2; and
Figure 4 is a cross sectional view of the screw according to the invention substantially taken along the line IV-IV of Figure 3.

### DESCRIPTION OF THE PREFERRED EMBODIMENTS

In the following disclosure, reference will be made to a preferred embodiment of the present invention which is illustrated, by way of an indicative but not limitative example of the possible variations of the invention.

The screws for intraosseous implants according to the present invention, generally indicated by the reference number 1, comprises a threaded portion 5 and a screw head 2.

The screw 1 comprises moreover an inner acetabulum 10, coaxial to the longitudinal axis of the screw, and so shaped as to allow it to be engaged both by suitable tools for applying the screw in a preformed bone cavity, and for a stable application on a bone stub on which the dental prostheses proper will be formed.

The threaded portion 5 is formed along the stem 4 of the screw 1, which ends with a rounded corner bottom 9.

The head 2 and stem 4 of the screw 1, on the top portion thereof not including the threaded part 5, are coupled by a truncated cone collet 3.

More specifically, the truncated cone collet 4 has a slanting angle with respect to the longitudinal axis of the screw 1, from 15° to 20°.

A particularly preferred value for that angle is of 17°.

The threaded portion 5 is provided, along its helical extension, with a plurality of gaps.

More specifically, the gaps of the helical extension of the threaded portion define finned helical surfaces which, as seen in plan in the direction of the longitudinal axis of the screw, comprise three longitudinal slots 6.

The longitudinal slots 6 are arranged at 120° from one another, with respect to a plane perpendicular to the axis of the screw 1.

Moreover, the threaded portion 5 is provided with a continuous "volute" 8, which represents the first volute or turn of the thread, as counted starting from the truncated cone collet 3.

Considering the outer characteristics of the material forming the screw 1 according to the present invention, it should be apparent that the head 2 and truncated cone collet 3 are subjected to a polishing operation.

The bottom portion of the screw 1, with respect to the truncated cone collet 3, is roughened by a sanding process, so as to provide a larger contact area with the sponge bone thereto it will be applied.

The screw 1 according to the present invention is preferably made of titanium.

The truncated cone collet 3 will provide the screw 1 with a greater stability property, thereby providing a great stability prosthesis, even in the case in which are used screws having a length smaller than that of conventional screws.

The gaps of the screw thread will allow pressure and organic liquids to be easily discharged, both during the application step of the screw 1 and in a subsequent step in which the screw 1 is embedded by the growing bone.

The continuous volute 8 will operate to close the bottom cavity of the screw 1 thereby preventing any bone piece to enter into said cavity.

The screw 1 for intraosseous implants according to the invention is of a fully submersed type.

Differently from other like implants, the subject screw must be applied so that the top portion of the neck 2 (on which the prosthesis portion or stub rests) is arranged embedded in the cortex to a depth of 0.1 - 1 mm under the bone surface.

The latter will regrowth in excess, radially along the perimeter of the neck 2 of the system, thereby filling the pervious space between the top portion of the neck and the gum mucose.

Upon stabilizing the implant, the excess ridge will be surgical cored in order to provide again a seat for introducing the prosthesis part or stub.

By the above disclosed implant procedure, the truncated cone collet 3 of the screw 1 will be arranged bridging the transition region between the cortex bone and the medullar bone.

In this region, the bone has good mechanical and resilient features and can resist against comparatively high loads.

From the above disclosure it should be apparent that the invention fully achieves the intended aim and objects.

## Claims

1. An improved screw for intraosseous implants, comprising a screw head and a screw stem provided with a threaded portion, characterized in that said screw comprises moreover a truncated cone collet coupling said screw stem and head, and that said threaded portion is provided, along the helical extension thereof, with a plurality of gaps.

2. An improved screw for intraosseous implants, according to Claim 1, characterized in that said gaps of said helical extension of said threaded portion define finned helicoidal surfaces on said threaded portion.

3. An improved screw for intraosseous implants, according to Claim 2, characterized in that said finned helicoidal surfaces, as seen in plan in the direction of the longitudinal axis of the screw, are provided with three longitudinal slots.

4. An improved screw for intraosseous implants, according to Claim 3, characterized in that said longitudinal slots are arranged at 120° from one another with respect to a plane perpendicular to the axis of said screw.

5. An improved screw for intraosseous implants, according to one or more of the preceding claims, characterized in that said threaded portion is provided with a continuous volute.

6. An improved screw for intraosseous implants, according to Claim 5, characterized in that said continuous volute is the first volute of the screw thread, as counted starting from said truncated cone collet.

7. An improved screw for intraosseous implants, according to one or more of the preceding claims, characterized in that said truncated cone collet is slanted with respect to the longitudinal axis of said screws with a slanting angle from 15° to 20°.

8. An improved screw for intraosseous implants, according to one or more of the preceding claims, characterized in that said head and truncated cone collet are subjected to a polishing working operation.

9. An improved screw for intraosseous implants, according to one or more of the preceding claims, characterized in that the bottom portion of the screw, with respect to said truncated cone collet, is roughened.

10. An improved screw for intraosseous implants, according to Claim 9, characterized in that said portion is roughened by sanding.

11. An improved screw for intraosseous implants, according to one or more of the preceding claims, characterized in that said screw is made of titanium.
